# EUROPEAN PATENT SPECIFICATION

(11) **EP 4 017 909 B1**
(45) Date of publication and mention of the grant of the patent: **15.11.2023**
(21) Application number: 20757349.4
(22) Date of filing: 20.08.2020
(51) Int. Cl.: C08J 11/24, C07C 67/52, C08L 67/02

(54) **METHOD AND REACTOR SYSTEM FOR DEPOLYMERISING A TEREPHTHALATE-POLYMER INTO REUSABLE RAW MATERIAL**
VERFAHREN UND REAKTORSYSTEM ZUR DEPOLYMERISATION EINES TEREPHTHALAT-POLYMERS IN EINEN WIEDERVERWERTBAREN ROHSTOFF
PROCÉDÉ ET SYSTÈME DE RÉACTEUR POUR LA DÉPOLYMÉRISATION D'UN POLYMÈRE DE TÉRÉPHTALATE EN UNE MATIÈRE PREMIÈRE RÉUTILISABLE

(30) Priority: 21.08.2019 NL 2023681
(43) Date of publication of application: 29.06.2022
(73) Proprietor: Ioniqa Technologies B.V., 5612 AR Eindhoven (NL)
(72) Inventor: WOLTERS, Joost Robert, 5583 CM Waalre (NL); DE HAAN, André Banier, 4741 SE Hoeven (NL); THIJS, Luuk, 5683 HG Best (NL)
(74) Representative: Arnold & Siedsma
(86) International application number: PCT/EP2020/073291
(87) International publication number: WO 2021/032821

(56) References cited:
- EP-A1- 0 723 951
- WO-A1-2016/010587
- JP-A- 2000 053 802
- JP-A- 2008 088 096
- US-A1- 2019 161 595
- GHAEMY M ET AL: "Depolymerisation of poly(ethylene terephthalate) fibre wastes using ethylene glycol", POLYMER DEGRADATION AND STABILITY, BARKING, GB, vol. 90, no. 3, 1 December 2005 (2005-12-01), pages 570-576, XP027766630, ISSN: 0141-3910 [retrieved on 2005-12-01]

## Description

### Field of Invention

The invention relates to a method for depolymerising a terephthalate polymer into reusable raw material. The invention further relates to a reactor system for depolymerising a terephthalate polymer into reusable raw material.

### Background

Terephthalate polymers are a group of polyesters comprising terephthalate in the backbone. The most common example of a terephthalate polymer is polyethylene terephthalate, also known as PET. Alternative examples include polybutylene terephthalate, polypropylene terephthalate, polyethylene isoterephthalate, poly pentaerythrityl terephthalate and copolymers thereof, such as copolymers of ethylene terephthalate and polyglycols, for instance polyoxyethylene glycol and poly(tetramethylene glycol), polyethylene oxide/polybutylterephthalate copolymers. PET is one of the most commons plastics and it is highly desired to recycle PET by depolymerization thereof into reusable raw material. One preferred way of depolymerization is glycolysis, which is preferably catalyzed. Typically, as a result of the use of ethylene glycol a reaction mixture comprising at least one monomer comprising bis (2-hydroxyethyl) terephthalate (BHET) may be formed. One example of a depolymerization is known from WO2016/105200 in the name of the present applicant. According to this process, the terephthalate polymer is depolymerized by glycolysis in the presence of a catalyst. At the end of the depolymerization process, water is added and a phase separation occurs. This enables to separate a first phase comprising the BHET monomer from a second phase comprising catalyst, oligomers and additives. The first phase may comprise impurities in dissolved form and as dispersed particles. The BHET monomer can be obtained by means of crystallization.

EP 0 723 951 A1 discloses a method for the depolymerization of PET into a bis(2- hydroxyethyl) terephthalate (BHET) of high purity through the reaction of waste PET with excess ethylene glycol in the presence of a transesterification catalyst at a temperature of 190-210 °C, and with which pure BHET can be recovered through controlled crystallization processes from an aqueous solution. EP 0 723 951 A1 does not disclose a BHET-dimer crystallization step prior to the BHET crystallization.

A high purity is required for reuse of the depolymerized raw material. As is well-known, any contaminant may have an impact on the subsequent polymerization reaction from the raw materials. Moreover, since terephthalate polymers are used for food and also medical applications, strict rules apply so as to prevent health issues.

While Applicant's process leads to a very high conversion of the terephthalate polymer and also facilitates separation of various additives from the monomer, it was nevertheless observed in further investigations that the crystallized BHET monomer is not colorless but rather yellowish or reddish. Such a monomer is not acceptable for reuse as monomer.

### Summary

Therefore, there is still a need for a process for depolymerising a terephthalate polymer into reusable raw material with a high purity, so as to be suitable for preparation of fresh terephthalate polymer.

There is also a need for a reactor system in which such process can be implemented.

According to a first aspect of the invention there is provided a method for depolymerising a terephthalate polymer into reusable raw material, the method comprising the steps of:
- depolymerizing a terephthalate polymer by using ethylene glycol into a depolymerised mixture comprising at least one monomer and at least one dimer, wherein said at least one monomer comprises bis (2-hydroxyethyl) terephthalate (BHET), and said at least one dimer comprises dimer of bis (2-hydroxyethyl) terephthalate (BHET-dimer);
- removing solid compounds from said depolymerised mixture to obtain a composition comprising the at least one dimer and the at least one monomer as solutes in a mixture of ethylene glycol and water;
- crystallizing BHET-dimer from said solution, thereby obtaining a mixture of BHET-dimer crystals and a first mother liquid;
- separating the crystallized BHET-dimer from the first mother liquid, and thereafter:
- forming BHET crystals from said first mother liquid, thereby obtaining a mixture of BHET crystals and a second mother liquid; and
- recovering the BHET crystals.

According to a second aspect of the invention, a reactor system is provided for performance of the method of the invention, as will be discussed in more detail below.

According to a third aspect of the invention, the invention relates to a solid composition, being polymerizable raw material obtained from depolymerization and comprising at least 70wt%, preferably at least 80wt% BHET dimer in crystallized form.

It has been understood by the inventors in the investigations leading to the present invention that the contamination of the crystallised BHET was due to simultaneous crystallisation of dimers and inclusion of contaminants in the solution from which the BHET was crystallised. It turned out that the dimer had a higher crystallisation temperature, at a given concentration of dimer in the solution, such that at conditions for optimal crystallisation of BHET monomer, the dimer crystallized vigorously with the effect of forming inclusions with any other contaminant, such as for instance iron-ions or iron oxide. By performing a dimer crystallisation step prior to the BHET crystallisation, the dimer concentration in the first mother liquid was significantly reduced and any vigorous dimer crystallisation (or even precipitation) during BHET crystallisation could be prevented. As a consequence, a crystalline BHET monomer product was obtained that meets the requirements of purity for subsequent polymerisation.

It has come as a surprise to the inventors that the dimer crystallisation and the BHET monomer crystallisation can be separated in time, such that the dimers can be effectively removed prior to crystallisation of the BHET monomer. It is however observed, that in one embodiment some crystallisation of the BHET monomer together with the dimer may occur. Such a co-crystallisation is not necessarily problematic, since the dimer may be returned into the depolymerisation stage for further depolymerisation into monomer, or the dimer may be transmitted to a further depolymerisation stage for conversion of the dimers and optionally any oligomers into monomers. In the embodiment wherein the dimers are further processed into monomers, it is deemed preferable that the crystallized dimers will be dissolved or dispersed into solvent, such as ethylene glycol, after removal of the first mother liquid. However, this is not deemed necessary. In an implementation, the separation of crystallized BHET-dimer is performed by means of a filtration unit and wherein further processing the separated crystallized BHET-dimer comprises removing the separated crystallized BHET-dimer from the filtration unit by means of a flushing stream, which comprises ethylene glycol.

In addition to the solution of said problem of coloration of the resulting monomer by means of the selective crystallisation of dimer, it has been found that the filtration time of the filtration of the BHET monomer is significantly reduced by means of reduction of the dimer content in the BHET crystalline material. With a reduction of the dimer content from 5% to 3.5%, the filtration time was reduced by 60%, and with a reduction of the dimer content from 5.0% to 1.5%, the filtration time was reduced with almost 75%. In addition, it was found that a reduced dimer content reduces the amount of liquid remaining behind in the residue, hence as part of the cake. Such a reduction of the liquid in the cake is relevant so as to reduce the time and effort needed for drying. Therefore, the invention relates in another aspect to a method of purifying a crude composition comprising bis (2-hydroxyethyl) terephthalate (BHET), said method comprises the steps of: (1) providing the crude composition that comprise BHET and dimer of bis (2-hydroxyethyl) terephthalate (BHET-dimer) as solutes in a mixture of ethylene glycol and water; (2) pre-treating said crude composition to obtain a first mother liquid having a BHET-dimer content of at most 3wt%, relative to the content of BHET; (3) forming BHET crystals from said first mother liquid, thereby obtaining a mixture of BHET crystals and a second mother liquid and; (4) separating the BHET crystals from said second mother liquid, preferably by means of filtration.

It is observed, for sake of clarity, that the pre-treatment is a crystallisation of BHET-dimer in an advantageous embodiment. However, it is not excluded that another pre-treatment is carried out, so as to arrive at conditions suitable for the crystallisation of BHET and the separation of formed crystals from the second mother liquid. For instance, the contents of the crude composition may be modified by dilution with solvent and/or the crude composition may be mixed with a stream comprising BHET with a lower amount of BHET-dimer so as to arrive at the specified BHET-content.

It is furthermore observed that the beneficial results were obtained for filtration of the BHET crystals from the second mother liquid. However, it is not excluded that an alternative separation technique such as centrifugation is used therefore. Also in centrifugation the effect is expected, as the wet BHET crystals, known as cake, will have a tendency to block fluid outlets in a centrifuge.

It is observed that the crude composition comprising BHET is more preferably equal to the composition comprising at least BHET and BHET-dimer as solutes. However, this is not necessarily the case, and the crude composition may be obtained from a different process, while it is preferably obtained from a depolymerisation process. It is for instance not excluded that the crude composition comprises solids, which are preferably removed in the pre-treatment step, and more preferably prior to the reduction of BHET-dimer content (for instance by crystallisation of BHET-dimer).

It is further observed for sake of completeness that the purification method as defined hereinabove, in one preferred embodiment, may be carried out in a reactor system comprising a dimer crystallization unit for crystallization of dimer from the crude , wherein remaining composition constitutes a first mother liquid, wherein a separator is arranged for separation of said crystalline dimer from said first mother liquid; a monomer crystallization unit arranged for crystallisation of monomer from said first mother liquid; and a monomer crystal recovering stage arranged downstream from the monomer crystallization unit. Further implementations of the reactor system will be apparent for the skilled person. It is for instance preferred that a reactor system comprises a controlling for controlling the process steps performed, including the settings of temperature, residence times, flow rates and the like, as is common in the art of chemical engineering.

In an alternative and highly interesting embodiment, the crystallized dimer is recovered, particularly in solid form, and further processed so as to serve as a raw material for fresh polymerisation. The resulting polymerizable raw material comprises at least 70wt% dimer, even without any further optimization. Such optimization of the crystallisation, as well as variation of the content of dimer in the composition to be crystallized, will give a dimer concentration that is preferably at least 80wt%, and most preferably significantly higher, such as at least 90wt%, at least 92wt%, at least 94wt% or even at least 95wt% dimer. The desired purity may be obtained in dependence of requirements from a customer. One advantage of the recovery of BHET-dimer as a product is that a higher ratio between the terephthalate polymer and the solvent (ethylene glycol) may be used in the depolymerisation stage.

In one further embodiment hereof, the solid composition further comprises both monomer and trimer of BHET. Preferably, the amount of monomer and trimer is equal based on mole%, within a margin of tolerance, which tolerance margin is for instance within 20% or even within 10%, said percentage being based on the amount of monomer. The content of monomer and trimer relative to dimer may be optimized and could be smaller than 5 mole%, between 5 and 10 mole% or even beyond 10mole%, said percentages being based on the molar amount of dimer. It has been understood that any precipitation - particularly crystallisation - of monomer and trimer is not detrimental for the solid composition, as long as the amounts (in moles) thereof are roughly equal. In such case, the overall average corresponds to dimer of BHET. As a consequence, it is feasible to supply a predefined amount of the solid composition for a subsequent polymerisation, notwithstanding variations in the exact amount of monomer, trimer and dimer.

The subsequent polymerisation may be performed on the basis of the dimer alone or in combination with monomer, such as the BHET obtained from the depolymerisation. Beneficial proportions between monomer and dimer in the subsequent polymerisation can be established separately by persons skilled in the field of terephthalate polymerisation. It is deemed an advantage of the use of BHET-dimer in the polymerisation, that less ethylene glycol is needed. As a consequence, less ethylene glycol is to be removed at the end of the polymerisation than when starting with BHET-monomer. The reduction in the use of ethylene glycol can be up to one third less, or even up to 50% less.

A specific advantage for polymerisation on the basis of a combination of monomer and dimer of BHET is that this combination has a lower melting point than pure BHET. The lowering of the melting point may be up to 30-40°C. As a consequence, the polymerisation may be carried out at a lower temperature. This is clearly efficient so as to reduce energy consumption. Moreover, the lower temperature may result in a lower reaction rate, this enabling better control over the polymerisation.

While it is preferred that the BHET-dimer, preferably in combination with BHET is used for the preparation of PET, it is not excluded that it is used for the preparation of other reactions, including the preparation of polyurethanes.

Preferably, the dimer concentration in the first mother liquid, thus after the dimer crystallisation, is at most 3 wt% relative to the monomer concentration therein, and more preferably at most 2.5wt% or even at most 2 wt%. These dimer concentrations in the first mother liquid have been found acceptable so as to arrive at a BHET crystallisation without forming inclusions. In an embodiment, the concentration of bis (2-hydroxyethyl) terephthalate in the first mother liquid is controlled to be in the range of 1 wt% - 30wt%, preferably in the range of 5 wt% - 25 wt% of the mother liquid at the start of the step of forming the BHET crystals. In one further embodiment, the BHET concentration is in the range of 10-20wt%.

Preferably, the crystallized BHET monomer includes iron-impurities in a concentration of at most 30 ppm and more preferably at most 25 ppm or even at most 20 ppm. Furthermore, the dimer concentration in the crystallized BHET monomer is preferably at most 3.0wt%, more preferably at most 2.5wt.% or even at most 2.0wt.%.. In experiments dimer concentrations of 1.5wt% have been achieved. The BHET monomer obtained in accordance with the invention is crystallised in a needle shape.

In an embodiment, said crystallizing BHET-dimer from said first mother liquid is performed substantially without forming crystals of BHET. The term 'substantially without forming BHET crystals' herein refers thereto that the amount of pure BHET crystal is less than 20wt%, preferably less than 15wt%, or even less than 10wt% as part of the dimer crystal. More preferably, the amount of BHET crystal in the dimer product is less than 5% of the BHET in the initial composition, more preferably less than 3% or even less than 2%. It may crystallize by itself or crystallize on dimer nucleus so as to form mixed crystals.

In an embodiment, crystallizing BHET-dimer comprises crystallizing at least 60 weight-% of the BHET-dimer, preferably at least 70 weight-%, relative to the initial total weight of the BHET-dimer in the solution. In experiments, at least 80weight% of the BHET dimer in the initial composition was crystallized, such as in the range of 83-86wt%.

The process conditions may be further controlled so as to achieve that the dimer crystallization occurs distinct from the BHET crystallisation. It has been found that it is feasible to grow the dimers to sizes in the micrometer range so that they can be separated via filtration with a filter with filter openings in the range of 5-20 µm, for instance 10-15 µm. If the dimer is transported for further depolymerisation, it appears preferable to keep the size relatively small. If the dimer is recovered, a larger size is deemed preferable. In the latter case, it is preferable to do a washing step using a second filter. Especially but not exclusively in the embodiment that the dimer is transported back for further depolymerisation, the use of a centrifuge instead of a filter is deemed an advantageous option. The phase remaining in the centrifuge will be a slurry rather than a solid mass, as in filtration, which solid mass can be transported and recycled more easily.

In a first embodiment, the step of BHET-dimer crystallisation is performed at a higher temperature than the step of forming BHET crystals. This has been found to be a reliable and practical embodiment for dimer concentrations in the solution of 5-15wt% relative to the BHET concentration. However, the same may also be applied for dimer concentrations exceeding said 15wt%, such as up to 25wt% or up to 30wt% or even higher. In an advantageous implementation, the crystallisation temperature T₂ of the BHET crystallization is in the range of 70°C - 30°C, preferably in the range of 60°C - 40°C. Preferably, the crystallisation temperature T₁ of the dimer crystallisation is higher than the BHET crystallisation temperature T₂ of at least 5°C, preferably at least 10 °C, more preferably at least 15 °C. The crystallisation temperature is herein defined as the temperature defined at the start of the crystallisation steps, thus typically at which the nucleation occurs. It is not excluded that the temperature changes or is actively modified during the crystallisation.

Preferably, the process conditions during the dimer crystallisation are controlled. Feasible control parameters include a concentration of bis (2-hydroxyethyl) terephthalate in the composition at the start of the step of forming the BHET crystals; and a volume ratio between water and ethylene glycol in composition during the step of forming the BHET crystals; and duration of the crystallisation, particularly by controlling the temperature within a predetermined range for a predefined residence time, such as 2 minutes to 120 minutes, preferably in the range of 5 minutes to 60 minutes.

In a second embodiment, an anti-solvent is added to the first mother liquid, prior to forming BHET crystals. The anti-solvent is preferably water or an aqueous solution, such as an aqueous salt solution. In this second embodiment, it is not necessary that the temperature of the BHET crystallisation is lower than the temperature of the dimer crystallisation. Rather, the solubility of the BHET is reduced by the addition of the anti-solvent. In a further variation, an anti-solvent is added to the first mother liquid and the temperature of BHET crystallisation is controlled to be lower than that of the dimer variation.

More generally, the process conditions may be controlled so as to control, the solution and/or the first mother liquid prior to the crystallisation step with respect to the concentration of the compound to the crystallized, a volume ratio between water and ethylene glycol and the control of the temperature during a predefined period.

In accordance with the invention, the formation of crystals (of dimer and of BHET) precedes a separation step in which the corresponding mother liquid is removed. The formation of crystals preferably occurs in a crystallisation reactor, such as a vessel. The separation step is carried out with filtration, centrifugation, a cyclone or another type of separator as known in the art.

In a first embodiment, the separation of crystalline dimer and first mother liquid occurs in the first separator. In other words, the composition comprising the dimer and monomer as solutes is recycled to the first separator by means of a feedback loop. This has the advantage that no separate separator is required for the dimer crystallisation. Particularly when the crystalline dimer is anyhow undesired as a product and to be further depolymerized to monomer, this seems practical. The dimer crystallisation unit could be arranged just downstream of the first separator, or in the feedback loop. In one further implementation, a switch can be foreseen, so as to choose between the feedback loop and the forward path towards the monomer crystallisation unit. Such a switch may be embodied as one or more valves, as known to the skilled person in the art of reactor engineering. The switch is typically, though not strictly necessarily, implemented to be under control of a control unit. Such an embodiment is deemed particularly advantageous, when the depolymerisation stage is implemented as a batch reactor. In another further implementation a recycling ratio is set between a flow rate into the feedback loop and a flow rate towards the monomer crystallisation unit. Herewith, the dimer concentration in the first mother liquid can be set and controlled. Preferably, the recycling ratio is controlled by a control unit and may be modified when needed. This implementation is deemed suitable both for batch operation and for continuous operation of the depolymerisation.

In a second embodiment, the separation of crystalline dimer and first mother liquid occurs in a second separator, that is arranged downstream of the dimer crystallisation unit and upstream of the monomer crystallisation unit. By use of a second separator, the crystalline dimer can be separated and where desired recovered in solid form, in a higher purity than when using the first separator thereto.

It is not excluded that the crystallisation reactor includes the separator, which is for instance activated after a predefined residence time. However, a separate separator is deemed preferable. In case that the crystals are to be recovered, a washing step is preferably carried out after the separation step. A band filter is deemed one practical arrangement for performing a separation step and a subsequent washing step. The characteristic size of the separation means can be chosen in dependence of the size of the generated crystals and a desired duration for the separation step. In an implementation, recovering the BHET crystals comprises separating the BHET crystals from the second mother liquid by means of filtration using a filter element.

The monomer is preferably recovered in solid form. It is deemed appropriate that the recovery is followed by a washing step and a drying step. Preferably, the BHET monomer crystals essentially consist of BHET, such as at least 95wt%, more preferably at least 96wt.% or even at least 97wt.%. More preferably, said BHET monomer crystals comprise at most 2.0wt% of dimer, at most 1.5wt% of dimer or even at most 1.0wt% of dimer. This has been found to reduce significantly the risk of contamination with cations.

In an embodiment, the depolymerizing step comprises controlling a molar ratio between said terephthalate polymer and ethylene glycol to control the molar ratio between BHET-dimer and BHET-monomer in the depolymerised mixture. Since the dimer is crystallized separately, the molar ratio between the dimer and the monomer of BHET can be increased. This corresponds to the use of less ethylene glycol, thus a higher ratio between terephthalate polymer and ethylene glycol. The depolymerizing step involves glycolysis, in which the ethylene glycol solvent is also a reactant to obtain BHET and BHET-dimer rather than for instance terephthalic acid that would be generated in hydrolysis. The depolymerising step is preferably catalysed by means of a catalyst. A variety of catalysts is known in the art for depolymerisation of PET. One group of catalysts is based on the use of iron and/or iron oxide, which could be Fe²⁺ or Fe³⁺. Such catalysts turn out to work but may lead to leakage of iron-ions into the solution to be crystallized. Preferably, use is made of a heterogeneous catalyst that can be recovered, at least partially, by means of a phase separation. More preferably, the heterogeneous catalyst is a particulate catalyst that is dispersed in the solvent (ethylene glycol). One most preferred example is the catalyst known from WO2017/111602A1.

Another example is the catalyst known from WO2018/143798A1**.**

In a further embodiment, an adsorption treatment is performed downstream of the further processing step in which the said solution is obtained. Such adsorption treatment serves to remove any dispersed particles in the solution and/or any dissolved organic compounds. Preferably, in accordance with one embodiment of the invention, the adsorption treatment is performed on the first mother liquid, thus downstream of the dimer crystallisation and separation. This turns out to increase the lifetime of the adsorption filter and to increase the overall product yield. In fact, it was observed in investigations that a significant amount of dimer adsorbed and thus was lost. Furthermore, the dispersed particles may act as nuclei for the crystallisation of the dimer, which further facilitates the dimer crystallisation. The adsorption treatment is for instance performed with active carbon as adsorption medium.

According to another aspect of the invention there is provided a reactor system for depolymerising a terephthalate polymer into reusable raw material, said reactor system comprising:
- a depolymerisation stage comprising at least one inlet for a stream of terephthalate polymer and a stream of ethylene glycol; wherein said depolymerisation stage is configured for depolymerizing the terephthalate polymer into a depolymerised mixture by using the ethylene glycol, wherein said depolymerised mixture comprises at least one monomer and at least one dimer, wherein said at least one monomer comprises bis (2-hydroxyethyl) terephthalate (BHET), and said at least one dimer comprises dimer of bis (2-hydroxyethyl) terephthalate (BHET-dimer);
- a first separator provided with an outlet, said first separator being configured for separation of solid compounds in the depolymerised mixture from a composition comprising the at least one dimer and the at least one monomer as solutes in a mixture of ethylene glycol and water;
- a dimer crystallization unit for crystallization of dimer from said composition, wherein remaining composition constitutes a first mother liquid, wherein a separator is arranged for separation of said crystalline dimer from said first mother liquid;
- a monomer crystallization unit arranged for crystallisation of monomer from said first mother liquid; and
- a monomer crystal recovering stage arranged downstream from the monomer crystallization unit.

This reactor system is configured for performance of the process of the invention. Typically each of the crystallisation units are embodied as at least one vessel with an inlet and an outlet. Preferably a controller is present for controlling process conditions in each of said vessels Sensors may be available thereto, as known to those skilled in the art. The crystallisation units, and the separator and recovering stage may be configured for batch operation or for continuous operation. Alternatively, the system is semi-continuous, in that the crystallisation units are of a batch type but the streams from the further processing stage and beyond are continuous. In this implementation, it is deemed preferable that a plurality of crystallisation units is arranged in parallel so as to load one crystallisation unit while performing the crystallisation treatment in another one.

An integrated reactor system has the advantage that heat loss is reduced to a minimum, which prevents unforeseen precipitation. It is a further advantage that a second mother liquid remaining after crystallisation of the BHET can be recycled for use in the depolymerisation stage. Thereto, it is preferably subjected to a distillation treatment so as to reduce a water content in the ethylene glycol. Preferred implementations thereof are described in the non-prepublished application NL2022037 in the name of Applicant, which is herein included by reference.

As set out above, the temperature in the dimer crystallisation stage may be higher than that in the monomer crystallisation stage. It is deemed preferable that the dimer crystallization stage comprises a temperature sensor for determining the temperature of the solution and that the monomer crystallization stage comprises a temperature sensor for determining the temperature of the first mother liquid. At least one controller unit of the reactor system is therein configured to control the first temperature T₁ of the solution and / or to control the second temperature T₂ of the first mother liquid, in response to a sensor signal of at least one of the first temperature sensor and the second temperature sensor. Alternatively or additionally, an inlet may be present to the monomer crystallisation stage and/or the dimer separation stage so as to add an anti-solvent such as water.

In one embodiment, water or an aqueous solution are added to the depolymerised mixture arriving from the depolymerisation stage prior to the first separator.. The water is more preferably water at a temperature of at least 85°C. This addition is preferably following by mixing of the added water or aqueous solution with the depolymerized mixture. The addition may occur within a depolymerisation reactor, in a separate mixing vessels, or in a vessel that is part of the first separator, especially but not exclusively when embodied as a centrifuge. The addition of the water and/or aqueous solution results in precipitation of at least part of the solid compounds that are thereafter removed in the first separator. Preferably the addition is controlled so as to generate a first phase and a second phase, wherein the first phase is the composition comprising monomer and dimer and the second phase comprises the solid compounds. The second phase may be a slurry or a suspension or the like. The composition may be a solution, but can alternatively be a dispersion, such as a colloidal solution including particulate matter that is not or hardly visible. The particulate matter therein preferably comprises nanoparticles and the like, for instance particles with a size smaller than 0.1, 0.5, 1 or 5 micrometer, as determined by sieving. The size limit may depend on the implementation of the first separator. It is deemed preferably that the concentration of particulate matter in the composition is low, for instance at most 5wt% or even at most 3wt%.

As discussed above, several embodiments are envisaged for the separation of the crystalline dimer from the first mother liquid. In a first major embodiment the first separator is used said separation of crystalline dimer and first mother liquid. In a second major embodiment, a second separator is present for said separation. In the first major embodiment, one may use a switch or a - predefined but modifiable - recycling ratio so as to achieve that the dimer-rich composition is recycled (before or after the crystallisation of dimer), while the remaining first mother liquid (relatively poor in dimer) is transported to the monomer crystallisation unit. Particularly but not exclusively in the second major embodiment, it is feasible to recover crystalline dimer in addition to or instead of generating a stream comprising dimer that is to be further depolymerized.

In one implementation, the second separator comprises an inlet for a flushing stream, which comprises ethylene glycol, wherein the separator for the dimer is configured to treat separated BHET-dimer crystals to obtain a stream comprising BHET-dimer and ethylene glycol. Said stream is preferably recycled to the depolymerisation stage. However, it is not excluded that such a stream would be transferred to a separate reaction vessel, in which reaction conditions are optimized for the depolymerisation of oligomers.

In a further implementation, the second separator comprises a filtration unit configured to separate the BHET-dimer crystals from the first mother liquid by means of filtration, and wherein the dimer separation stage is configured to transport the flushing stream through the filtration unit in a flow direction opposite to a flow direction of the first mother liquid of the filtration step.

In an embodiment, the monomer crystal recovering stage is further configured for washing the separated BHET crystals by means of a wash stream and is configured for drying the separated BHET crystals.

In an embodiment, the monomer crystal recovering stage comprises a filtration unit configured to separate the BHET crystals from the second mother liquid by means of filtration, and wherein the filtration unit is configured to carry out the washing of the separated BHET crystals inside the filtration unit.

Advantageously, an adsorption means is present and arranged downstream of the second separator. It has been understood that this arrangement results in a longer lifetime of the adsorption means, such as a column or a filter.

It is observed for clarity that any of the embodiments discussed hereinabove and/or hereinafter with reference to the figures or in the context of the examples or as defined in the dependent claims with respect to one aspect of the invention is also applicable and deemed disclosed in relation to any other aspect of the invention, which aspects are further defined in the claims as filed.

### Brief description of the figures

The above and other advantages of the features and objects of the invention will become more apparent and the invention will be better understood from the following detailed description when read in conjunction with the accompanying drawings, in which:
Fig. 1 illustrates schematically a first embodiment of the reactor system of the invention,
comprising a dimer crystallisation stage and a monomer crystallisation stage;
Fig. 2 schematically illustrates a second embodiment of the reactor system of the invention;

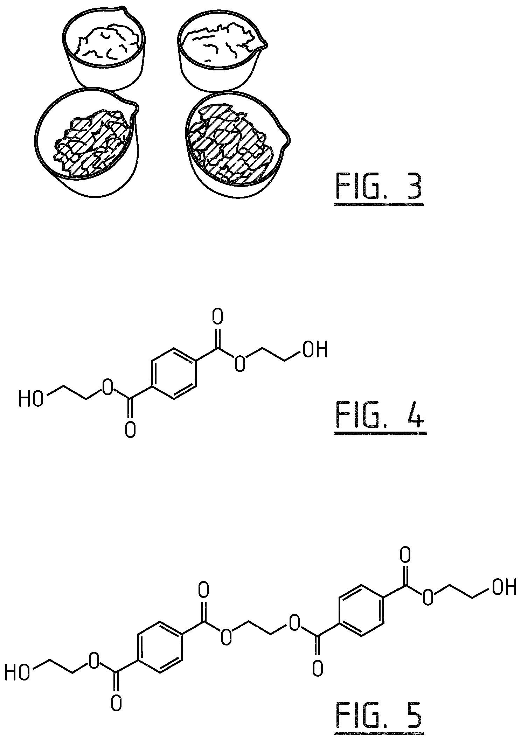

Fig. 3 shows a picture of recovered BHET crystals (after a washing step), with or without containing Fe2+ or Fe3+ elements;
Fig. 4 shows a molecular structure of BHET;
Fig. 5 shows a molecular structure of BHET-dimer.
Fig. 6 shows a HPLC of BHET-dimer

### Description of embodiments

The accompanying drawings are used to illustrate presently preferred non-limiting exemplary embodiments of devices of the present invention. The figures are not drawn to scale. The same reference numerals in different figures refer to equal or corresponding elements.

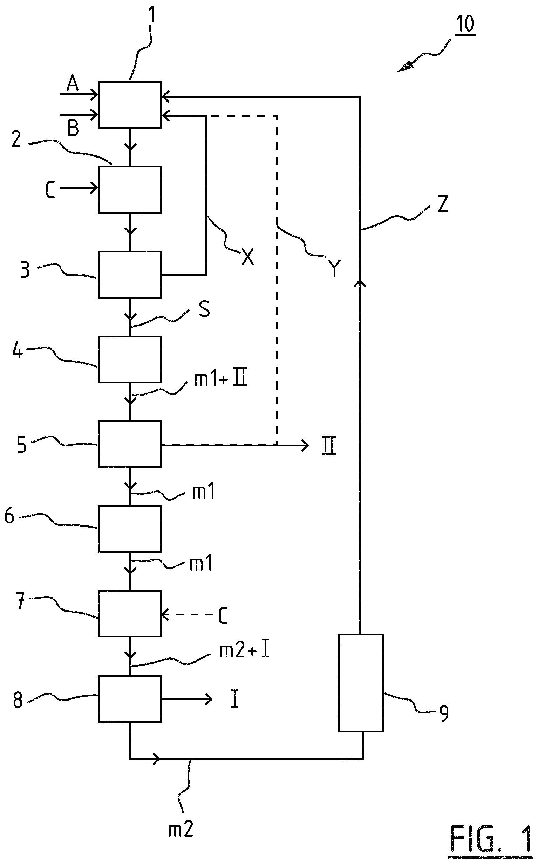

Figure 1 illustrates schematically a first embodiment of the reactor system 10 of the invention. The shown reactor system 10 essentially comprises a depolymerisation reactor 1, three units 2, 4 and 7 in which a phase separation occurs and four separation means 3, 5, 6, 8 for a solid-liquid separation. Feedback streams X, Y and Z are indicated which recycle catalyst, intermediate products such as oligomers and solvent, in particularly ethylene glycol. It will be understood that the Fig. 1 is a highly schematic illustration and that any variations or amendments are not excluded.

The reactor system 10 is provided with an input stream A comprising polymeric material. Preferably, this polymeric material has been pre-separated so that at least the bulk thereof is the terephthalate polymer for depolymerisation, more particularly PET. The input steam may be in the solid form, such as in the form of flakes. However, it is not excluded that the input stream is in the form of a dispersion or even a solution.

The input stream A goes into the depolymerisation reactor 1. Other streams entering this depolymerisation reactor include a recycled stream Z of solvent, such as ethylene glycol, a recycled stream X of oligomers and catalyst. A recycled stream Y of dimers is optional and therefore indicated as a dashed line. Further fresh ingredients, such as fresh catalyst and fresh solvent are indicated as input stream B. The input streams A, B, X, Y, Z may be arranged as individual inlets or may be combined into one or more inlets. The depolymerisation reactor 1 may be of a batch type or a continuous type. While it is indicated as a single reactor, it is not excluded that a combination of reactor vessels is used, such as the combination of a tank reactor and a plurality of plug flow reactors as disclosed in WO2016/105200A1. Also a plurality of vessels may be arranged in parallel within one unit as indicated in Figure 1. While not indicated, it will be understood that the reactor system 10 is provided with a controller and that sensors may be present as well as valves for setting flow rates into the reactor and for setting residence times in the reactor. Furthermore, the reactors 1, 2, 4 and 7 will be provided with heating means and/or other temperature regulation means so as to prevent deviations from predefined temperatures and other variables.

Following the depolymerisation, the depolymerised reaction mixture is pumped to a cooling vessel 2, which is provided with an inlet for water C. The water C may alternatively be provided as an aqueous solution. It is not excluded that one or more further additives are added thereto, so as to facilitate the phase separation. The cooling vessel 2 serves to cool down the depolymerized mixture from a depolymerisation temperature, typically in the range of 160-200°C, to a processing temperature, for instance around 100°C. The water may contribute to the cooling process. As a consequence of the addition of water, a two-phase mixture is generated, with a first phase of monomer and dimer as solutes in a mixture of ethylene glycol and water, and a second phase comprising oligomers, catalyst, additives. The two-phase mixture is thereafter separated in the first separator 3, for instance a centrifuge. The second phase is thereafter recycled to the depolymerisation reactor as stream X. While the separator 3 and the cooling vessel 2 are indicated herein as separate units, it is not excluded that the cooling step would occur within the first separator 3. Alternatively, the depolymerisation reactor 1 and the cooling vessel 2 may be physically a single unit, particularly in case of using a batch process.

The first phase leaving the first separator 3 is also referred to as a solution S in the context of the present invention. Rather than a pure solution, the solution S may be a colloidal solution or a dispersion. The solution is transferred to the dimer crystallisation stage 4. This is preferably a mixing vessel that is provided with temperature regulation means for bringing and keeping the crystallizing solution at a predefined temperature, for instance in the range of 50-70 °C, such as 57-64°C, for instance 60°C. The exact temperature will depend on the concentration of the dimer, the ratio between water and ethylene glycol and the desired residence time. After a sufficient degree of crystallisation, the resulting combination of first mother liquid M1 and dimer crystals II is transferred to a second separator 5. A centrifuge, a cyclone and a filtration unit can be used for the second separator 5, and other separators are not excluded. The implementation will furthermore depend on any desire to collect the dimer crystals II as a separate product or merely for recycling to the depolymerisation reactor 1. When recycled, this preferably occurs in the form of a dispersion or solution in ethylene glycol. The implementation may also depend on the amount of dimer crystal that is recovered. While traditionally the BHET monomer is considered as the starting point for fresh polymerisation of terephthalate polymer, the ability to recover dimer enables the use thereof in the polymerisation. As a consequence, it becomes feasible to control the depolymerisation so as to generate significantly more dimer than traditionally intended. For instance, traditionally, the concentration of dimer is in the range of 5-10wt% of the amount of BHET. In accordance with one embodiment of the invention, in which the dimer is recovered as a product, the depolymerisation may be controlled so as to arrive at a dimer concentration of 15-50wt% relative to the amount of BHET.

The first mother liquid M1 is thereafter passed to an adsorption treatment 6, which is preferably arranged as a column or a filter, such as an active carbon column. This adsorption treatment serves to eliminate any solid particles in the mother liquid. Placing the adsorption treatment downstream of the dimer crystallisation prevents the adsorption of dimer thereon. In order to prevent crystallisation of BHET during such adsorption treatment, it is deemed beneficial that the adsorption treatment is arranged within a room held at sufficiently high temperature. It is not excluded that the first mother liquid will be heated prior to passing through the adsorption treatment and/or that the first mother liquid is diluted with further ethylene glycol, so as to prevent any undesired crystallisation of BHET. It is not excluded that the adsorption column 6 is arranged at a different position within the reactor system 10.

Downstream thereof, the BHET is crystallized in the BHET crystallisation stage 7 and recovered in a separator 8 as monomer product I. Rather than or in addition to lowering the temperature relative to the dimer crystallisation stage 4, water may be added to the first mother liquid (as indicated in the figure by means of the dashed line C). This will reduce the solubility of BHET and enable crystallisation and a higher temperature. Upon the crystallisation of the BHET, the first mother liquid M1 is transformed into the second mother liquid M2, which is recycled subsequent to the recovery of the BHET monomer product I. The recycling suitably includes processing of the mother liquid M2 to reduce its water content. This occurs in the processing stage 9, which preferably includes at least one distillation column. The resulting upgraded ethylene glycol is returned to the depolymerisation reactor 1 as stream Z.

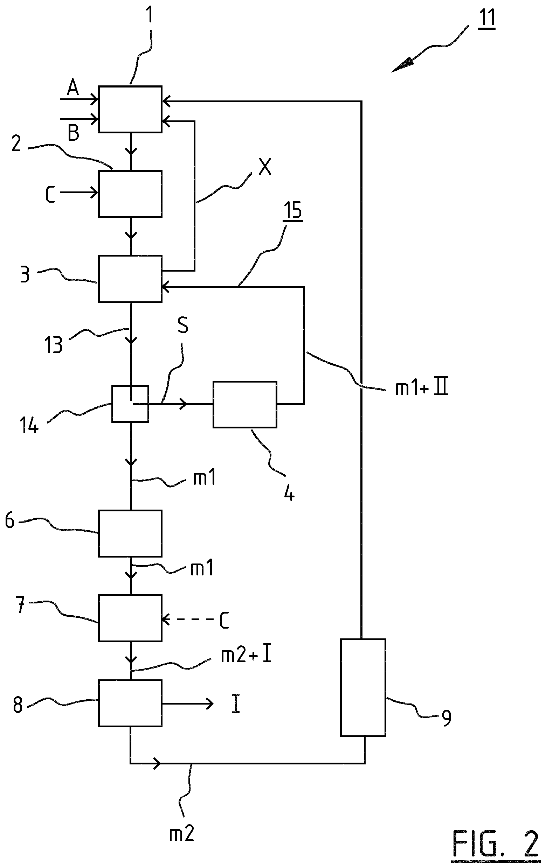

By means of the process of the invention, it has turned out feasible to arrive at a BHET monomer product I that is white and free of major contaminants. The result relative to a comparative example is shown in Figure 2 and will be discussed in more detail in the examples below. Figure 2 shows a photograph of four samples including 50 ppm iron (Fe)-ions, said iron ions being either Fe²⁺ or Fe³⁺. Iron is a common part of a catalyst. In the presence of dimer, the BHET crystals turned yellow to reddish. In the absence of dimer, the BHET crystals were white.

Fig. 2 schematically shows a second embodiment of the reactor system 11 of the invention. In contrast to the first embodiment shown in Fig. 1, the reactor system 11 according to the second embodiment does not include a second separator 5. Rather, the first separator 3 is used for separation of the dimer crystals II and the first mother liquid M1. In the illustrated embodiment a controlled switch 14 is present in the output stream 13 exiting the first separator 3. This control switch 14 is configured so that the solution S flows into a feedback loop 15 which comprises the dimer crystallisation unit. The switch is further configured so that the first mother liquid M1 flows towards the adsorption means 6. The control switch 14 is suitably under control of a control unit (not shown). Rather than in the output stream 13 at a location downstream from the first separator 3, the switch 14 may be arranged at the output of the first separator 3. The switch 14 may be configured as a pair of valves, so as to selectively open or close the feedback loop 15 or rather the path to the monomer crystallisation unit. The switch may further be configured as one or more devices for setting a flow rates. A combination hereof is feasible as well. The feedback loop 15 may end up in the cooling vessel 2 rather than in the first separator 3.

Further variations may be envisaged by a skilled person. It is for instance feasible that the recycling of one or more of the streams X, Y, Z comprises a (further) purification step, heating or cooling step. It is not excluded that the streams X, Y and/or Z are merged prior to the entry into the depolymerisation stage, or even that the streams X and Y are combined with the second mother liquid M2, so as to pass through the processing stage 9.

### Examples

### Example 1

A first experiment was carried out in which the initial composition was contaminated with 50 ppm iron, either Fe²⁺ or Fe³⁺. In this first, preliminary experiment, a single crystallisation was carried out to crystallize BHET, without any preceding separate crystallisation of BHET dimer. The crystallisation temperature was 50°C, as set with a heating bath. After crystallisation, the mother liquid was removed by filtration and the crystalline material were dried. Compositions were prepared that contained either dimer-rich BHET (with 8wt% dimer relative to the BHET), or pure BHET. The compositions all included 230 gram demineralized water and 230 gram ethylene glycol and 40 gram BHET or dimer-rich BHET. Results are shown in Table 1.

Surprisingly, the crystals including BHET-dimer had a yellow colour, whereas the pure BHET was white. As such, it turns out that the presence of BHET dimer results in undesired colorisation of the BHET crystals.

**Table 1 - results of a first preliminary experiment**

| Example | BHET-dimer | Fe | Colour |
|---|---|---|---|
| | [wt% w.r.t. BHET] | [type ppm]-w.r.t. [BHET] | |
| I | 8.1 | 50 ppm (Fe²⁺) | Yellow |
| II | 8.1 | 50 ppm (Fe³⁺) | Yellow |
| III | 0 | 50 ppm (Fe²⁺) | White |
| IV | 0 | 50 ppm (Fe³⁺) | White |

### Protocol of Example 2 and further

A composition is prepared for crystallisation experiments. This composition comprises 40 g dimer rich BHET, 230 g of demineralized water and 230 g of ethylene glycol (EG). The dimer-rich BHET is obtained from depolymerisation experiments in which PET is depolymerised by means of catalysed glycolysis and subsequent removal of oligomers and catalyst by addition of water and a centrifuge treatment. Use was made of the catalyst specified in WO2017111602, which is included herein by reference. However, the use of alternative catalysts is not excluded. In order to ensure that the dimer-rich BHET is sufficiently pure, it is treated by means of active coal, de-ionization resin several times - a treatment feasible on labscale for experiments, not for industrial production. The dimer-rich BHET used in the experiments typically contains about 8 wt% dimer, relative to the amount of BHET, unless otherwise specified. It typically also comprises other depolymerisation products from PET in small amounts (less than 1% per contamination), including BHET trimer, potentially BHET tetramer (in less quantities), isoBHET (i.e. an isomer to BHET) and BHEET (a monomer including more units ethylene glycol than BHET). Iron ions were added as a specific contamination. The iron dose used in the experiment was 50 ppm.

The ingredients of the composition are added to a 500 ml flask, provided with a lid. Subsequently, a (magnetic) stirring bean is added to the flask. The flask is placed on a heating plate and heated to approximately 90 °C, so as to dissolve all solid material (i.e. the dimer-rich BHET). The closed flask is then placed in a heating bath that has been prepared to have a temperature of 60°C. It remains therein for 20 minutes, in order to selectively crystallize the dimer.

The resulting mother liquid with the BHET-dimer crystals is separated by means of a Buchner funnel setup using filtration paper with openings of 12 - 15 µm. The Buchner funnel setup has been pre-heated, so as to avoid extra crystallisation of BHET. Thereto 100g demineralized water was boiled and poured through the Buchner funnel setup directly prior to the - slow- addition of the dimer crystals and the mother liquid. A magnet is held against the bottom of the flask, so as to ensure that the stirring bean remains in the flask. The filtrated mother liquid is a clear colorless liquid, that will over time start to crystallize. The residue in the Buchner funnel is a white cake. This residue is thereafter dried overnight in an oven.

The filtrated mother liquid is then re-heated to 80°C, so as to ensure that all crystals are redissolved. This is done for sake of the experiment on labscale. The mother liquid is thereafter crystallized by reducing the temperature in a controlled manner, for instance by using a series of heating baths at mutually decreasing temperatures. Thereafter, the second mother liquid containing BHET crystals is filtrated over another Bucher funnel setup, using filtration paper with openings of 12-15 µm. This filtration was carried out at room temperature. The resulting BHET is a white cake. The cake is washed with demineralized water. The washed cake is dried.

### Example 2

An experiment was carried out to evaluate dimer-crystallization under different conditions. The crystallisation temperature (i.e. of the bath) was 60°C in all cases. As shown in Table 2, it was found that the dimer-crystallisation results in a dimer content of approximately 2.0 wt.% (i.e. 1.5-2.5wt%), relative to the BHET, in the first mother liquid used for crystallisation of BHET.

**Table 2 - dimer crystallisation**

| | BHET | BHET-dimer | Time | BHET | BHET-dimer |
|---|---|---|---|---|---|
| | [wt% w.r.t. total weight] | [wt% w.r.t. BHET] | [minute] | [wt% w.r.t. total weight] | [wt% w.r.t. BHET] |
| 1 | 7.41 | 8.0 | 10 | 7.1 | 1.9 |
| 2 | 7.36 | 8.0 | 20 | 7.0 | 1.9 |
| 3 | 7.65 | 4.0 | 10 | 7.2 | 2.2 |
| 4 | 7.68 | 4.0 | 20 | 7.3 | 2.3 |

### Example 3

The pre-treatment was carried out as described in the protocol above, starting with a contamination of 50 ppm iron. Measurements were made to identify amounts of crystal and amounts of Fe-contamination. A comparative experiment was carried out, in which the dimer-rich BHET contains merely 5wt% dimer, relative to the BHET, but without separate dimer-crystallisation step. Furthermore, a reference was included, with 0% dimer. Table 3 shows the BHET, iron and dimer contents for the initial compositions, the second mother liquid and the obtained BHET. It also shows the obtained amount of BHET. It can be concluded from these data, that the dimer crystallization significantly reduces the dimer content in the BHET crystals, and that the iron content reduces in a corresponding manner.

**Table 3 - dimer and iron content**

| | Reference | Invention | comparative |
|---|---|---|---|
| Initial composition (weight, g) | 481 g | 484 g | 482 g |
| BHET (wt% total weight) | 9.2% | 8.6% | 8.1% |
| Dimer (wt% w.r.t. BHET) | 0% | 10.1% | 5.1% |
| Fe (ppm) | 50 ppm | 50 ppm | 50 ppm |
| | | | |
| Composition after pretreatment (if any) | - | 480 g | - |
| BHET (wt% total weight) | | 8.4% | |
| Dimer (wt% to BHET) | | 2.0% | |
| | | | |
| 2^{nd} mother liquid (after BHET crystallisation) | 397 g | 394 g | 399 g |
| BHET (wt% total weight) | 0.97% | 0.99% | 0.90% |
| Dimer (wt% to BHET) | 0.03% | 1.0% | 4.7% |
| Fe (ppm) | 59 ppm | 56 ppm | 54 ppm |
| | | | |
| BHET crystal wet | 46 g | 48 g | 62 g |
| BHET crystal dry | 31 g | 31 g | 31 g |
| Weight removal during drying | 15 g | 17 g | 31 g |
| Dimer (wt%) | 0% | 1.5% | 5.1% |
| Fe (ppm) | 6 ppm | 20 ppm | 53 ppm |

### Example 4

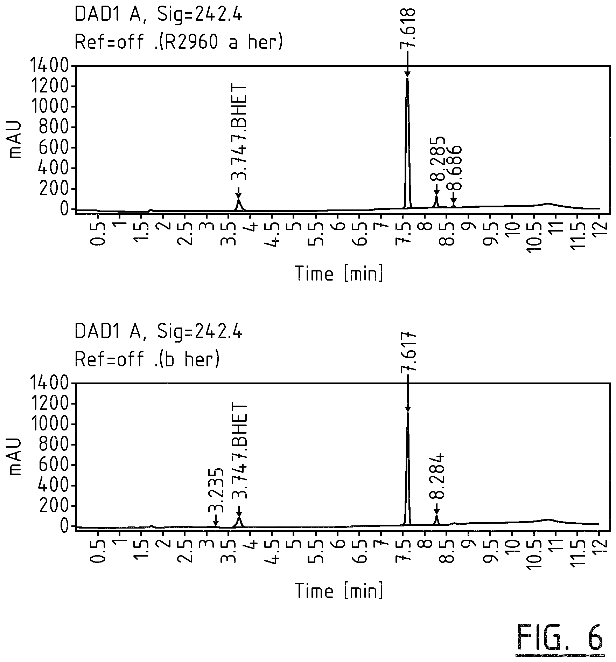

Figure 6 shows HPLC-diagrams of the BHET dimer formed in accordance with the protocol specified above. The identification was carried out in duplo. The peak at 3.75 minutes is representative of BHET. The peak at 7.62 minutes is representative for the dimer. The peak at 8.28 minutes indicates trimer of BHET. The peak at 8.69 minutes is assumed to correspond to BHET tetramer. The relative contents of the different compounds (in wt%) are shown in Table 4:

**Table 4- contents of BHET, BHET dimer and further oligomers in the dimer crystal, all in wt%**

| Compound | Time (min) | First HPLC | Second HPLC | Average |
|---|---|---|---|---|
| BHET | 3.75 | 11.9 | 11.8 | 11.9 |
| BHET-dimer | 7.62 | 77.0 | 76.5 | 76.7 |
| BHET-trimer | 8.28 | 7.7 | 11.7 | 9.7 |
| BHET-tetramer | 8.69 | 3.4 | 0 | 1.7 |

It is observed that the values for BHET are higher than what is obtained when using a calibration standard for BHET. Then the average value ends up at 10.4wt%, with a standard deviation of 0.7. It is furthermore observed that these contents are the result of a first experiment that was not optimized. Beyond that, it is known that the amount of dimer in the composition relative to the amount of BHET monomer may be modified, by means of depolymerisation conditions, such as the ratio between ethylene glycol and polymer (PET). As a consequence, the monomer and dimer contents of the obtained dimer crystal may well vary. Effectively, without further optimization of the crystallisation and without major change in the composition to be crystallized, the BHET content will be in the range of 8-12wt% (based on the calibration) and the dimer content in the range of 70-85wt%. Using different settings, the monomer content may well change in the range of 5-15wt%, and the dimer content ranging from 67 to 90wt%. When the crystallisation is further optimized for obtaining a pure dimer, for instance by optimizing the crystallisation temperature and change therein, the dimer content may well exceed the 90wt%, for instance 90-95wt% or even above the 95wt%, such as up to 97wt.%. As discussed before, the dimer content in the composition may further be optimized such that the amount of BHET and BHET trimer is equal within a range of tolerance (such as up to 3wt%). More particularly, said amounts are equal with respect to the molar quantities, i.e. that the dimer crystal for instance comprises 0.1-0.2 mole BHET and 0.1-0.2 mole trimer per mole of dimer.

### Example 5: Filtration process BHET

The duration of the filtration process of the second mother liquid including BHET crystals was investigated. In each case, the same filtration paper was used, having openings of 12-15 µm. A comparison was made between different compositions, having a different concentration of dimer in the BHET crystals and in the second mother liquid.. The iron content in the second mother liquid was roughly the same for all compositions. Table 5 shows the results.

**Table 5 - filtration time in dependence of dimer concentration.**

| Composition | A | B | C | D |
|---|---|---|---|---|
| 2^{nd} mother liquid (after BHET crystallisation) | 397 g | 394 g | 394 g | 399 g |
| BHET (wt% total weight) | 0.97% | 0.99% | 0.94 | 0.90% |
| Dimer (wt% to BHET) | 0.03% | 1.0% | 4.8 % | 4.7% |
| BHET crystal wet | 46 g | 48 g | 61g | 62 g |
| BHET crystal dry | 30.8 g | 30.8 g | 31.0 g | 30.8 g |
| Weight removal during drying | 15 g | 17 g | 30 g | 31g |
| Dimer (wt%), after drying | 0% | 1.5% | 3.5% | 5.1% |
| Filtration time (seconds) | 16 | 46 | 69 | 172 |

The filtration time was found to increase significantly, in dependence on the dimer concentration. Several conclusions can be drawn from the data in this table. First of all, the weight of the wet BHET crystal (typically in the form of a cake) significantly depends on the dimer concentration. Herein, the cake weight for compositions A and B is similar and the cake weight for the compositions C and D is similar, and almost twice that of compositions A and B. It seems that this cake weight is mostly dependent on the dimer concentration in the second mother liquid, which is almost equal for compositions C and D. Secondly, the filtration time is highly dependent on the dimer concentration. Here, the dimer concentration in the BHET crystal seems to be very important. The filtration time for the composition B is comparatively long. In further experiments, however, the filtration time for mother liquids and BHET crystals obtained with the invention could be further reduced to less than 30 seconds.

## Claims

1. A method for depolymerising a terephthalate polymer into reusable raw material, the method comprising the steps of:
a) depolymerizing a terephthalate polymer by using ethylene glycol into a depolymerised mixture comprising at least one monomer and at least one dimer, wherein said at least one monomer comprises bis (2-hydroxyethyl) terephthalate (BHET), and said at least one dimer comprises dimer of bis (2-hydroxyethyl) terephthalate (BHET-dimer);
b) removing solid compounds from said depolymerised mixture in a first separator to obtain a composition comprising the at least one dimer and the at least one monomer as solutes in a mixture of ethylene glycol and water;
c) crystallizing BHET-dimer from said solution , thereby obtaining a mixture of BHET-dimer crystals and a first mother liquid;
d) separating the crystallized BHET-dimer from the first mother liquid, and thereafter:
e) forming BHET crystals from said first mother liquid, thereby obtaining a mixture of BHET crystals and a second mother liquid; and
f) recovering the BHET crystals.

2. The method according to claim 1, wherein after the step of BHET-dimer crystallisation and before the step of forming crystals of BHET an anti-solvent is added to the first mother liquid to enhance the forming of crystals of BHET from the first mother liquid.

3. The method according to any one of the preceding claims, the step of crystallizing BHET-dimer comprises controlling at least one of:
i. a concentration of BHET-dimer in the solution at the start of the step of crystallizing BHET-dimer; and
ii. a volume ratio between water and ethylene glycol in the solution during the step of crystallizing BHET-dimer; and
iii. controlling the solution at a first temperature T₁ for a period in the range of 2 minutes to 120 minutes, preferably in the range of 2 minutes to 60 minutes.

4. The method according to any of the preceding claims, wherein crystallizing BHET-dimer from said solution is performed substantially without forming crystals of BHET.

5. The method according to any one of the preceding claims, wherein the crystallized BHET is recovered in solid form, and wherein preferably the method further comprises at least one of the steps of washing the BHET crystals and drying the BHET crystals.

6. The method according to claim 5, wherein the separating of crystallized BHET-dimer is performed by means of a filtration unit and wherein further processing the separated crystallized BHET-dimer comprises removing the separated crystallized BHET-dimer from the filtration unit by means of a flushing stream, which comprises ethylene glycol.

7. The method according to claim 6, wherein said stream is processed to convert the BHET-dimer into BHET.

8. The method according to any one of the preceding claims, wherein the depolymerizing step comprises controlling a molar ratio between said terephthalate polymer and ethylene glycol to control the molar ratio between BHET-monomer and BHET-dimer in the depolymerised mixture, wherein preferably said molar ratio is controlled to obtain at least 10wt% of dimer.

9. The method according to any one of the preceding claims, wherein the first mother liquid is subjected to an adsorption treatment to remove dissolved BHET-dimer from the first mother liquid after separating the crystallized BHET-dimer from the first mother liquid and before the formation of BHET crystals.

10. The method according to any one of the preceding claims, wherein the depolymerizing step is carried out by means of a catalyst comprising element Fe, and wherein the depolymerized mixture comprises element Fe at a concentration of at least 5 ppm with respect to the weight concentration of bis (2-hydroxyethyl) terephthalate (BHET) in the depolymerized mixture.

11. A reactor system for depolymerising a terephthalate polymer into reusable raw material, said reactor system comprising:
- a depolymerisation stage comprising at least one inlet for a stream of terephthalate-containing polymer and a stream of ethylene glycol; wherein said depolymerisation stage is configured for depolymerizing the terephthalate-containing polymer into a depolymerised mixture by using the ethylene glycol, wherein said depolymerised mixture comprises at least one monomer and at least one dimer, wherein said at least one monomer comprises bis (2-hydroxyethyl) terephthalate (BHET), and said at least one dimer comprises dimer of bis (2-hydroxyethyl) terephthalate (BHET-dimer);
- a first separator provided with an outlet, said first separator being configured for separation of solid compounds in the depolymerised mixture from a composition comprising the at least one dimer and the at least one monomer as solutes in a mixture of ethylene glycol and water;
- a dimer crystallization unit for crystallization of dimer from said composition, wherein remaining composition constitutes a first mother liquid, wherein a separator is arranged for separation of said crystalline dimer from said first mother liquid;
- a monomer crystallization unit arranged for crystallisation of monomer from said first mother liquid; and
- a monomer crystal recovering stage arranged downstream from the monomer crystallization unit.

12. Solid composition comprising at least 70wt% dimer of bis (2-hydroxyethyl) terephthalate (BHET) in crystalline form, wherein the solid composition preferably comprises at least 80wt% dimer of BHET.

13. A method of purifying a crude composition comprising bis (2-hydroxyethyl) terephthalate (BHET), said method comprises the steps of
a) Providing the crude composition that comprises BHET and dimer of bis (2-hydroxyethyl) terephthalate (BHET-dimer) as solutes in a mixture of ethylene glycol and water;
b) Pre-treating said crude composition to obtain a first mother liquid having a BHET-dimer content of at most 3wt%, relative to the content of BHET;
c) forming BHET crystals from said first mother liquid, thereby obtaining a mixture of BHET crystals and a second mother liquid and;
d) separating the BHET crystals from said second mother liquid, preferably by means of filtration; and
e) optionally further comprising the step of drying the separated BHET crystals.

14. The method as claimed in claim 13, wherein the pre-treatment of the crude composition comprises crystallisation of BHET-dimer, and preferably separation of the crystallized BHET-dimer from the remaining first mother liquid.

15. The method as claimed in any one of claims 13-14, wherein said BHET-dimer-content in the first mother liquid is at most 2.0wt.%, relative to BHET.

## Patentansprüche

1. Ein Verfahren zur Depolymerisation eines Terephthalatpolymers zu wiederverwendbarem Rohmaterial, wobei das Verfahren die folgenden Schritte aufweist:
a) Depolymerisieren eines Terephthalatpolymers unter Verwendung von Ethylenglykol zu einem depolymerisierten Gemisch, das zumindest ein Monomer und zumindest ein Dimer aufweist, wobei das zumindest eine Monomer Bis(2-hydroxyethyl)terephthalat (BHET) umfasst und das zumindest eine Dimer ein Dimer von Bis(2-hydroxyethyl)terephthalat (BHET-Dimer) umfasst;
b) Entfernen von festen Verbindungen aus dem depolymerisierten Gemisch in einem ersten Separator, um eine Zusammensetzung zu erhalten, die das zumindest eine Dimer und das zumindest eine Monomer als gelöste Stoffe in einer Mischung aus Ethylenglykol und Wasser aufweist;
c) Auskristallisieren des BHET-Dimers aus der besagten Lösung, wodurch ein Gemisch aus BHET-Dimerkristallen und einer ersten Mutterflüssigkeit erhalten wird;
d) Abtrennen des kristallisierten BHET-Dimers von der ersten Mutterflüssigkeit, und danach
e) Bilden von BHET-Kristallen aus der ersten Mutterflüssigkeit, wodurch ein Gemisch aus BHET-Kristallen und einer zweiten Mutterflüssigkeit erhalten wird; und
f) Rückgewinnen der BHET-Kristalle.

2. Das Verfahren nach Anspruch 1, wobei, nach dem Schritt der BHET-Dimer-Kristallisation und vor dem Schritt des Bildens von BHET-Kristallen, der ersten Mutterflüssigkeit ein Anti-Lösemittel zugesetzt wird, um das Bilden von BHET-Kristallen aus der ersten Mutterflüssigkeit zu fördern.

3. Das Verfahren nach einem der vorherigen Ansprüche, wobei der Schritt der BHET-Dimer-Kristallisation das Steuern von zumindest einer der folgenden Größen umfasst:
i. einer Konzentration des BHET-Dimers in der Lösung zu Beginn des Schritts der BHET-Dimer-Kristallisation, und
ii. eines Volumenverhältnisses zwischen Wasser und Ethylenglykol in der Lösung während des Schritts der BHET-Dimer-Kristallisation; und
iii. das Einstellen der Lösung auf eine erste Temperatur T1 für einen Zeitraum im Bereich von 2 Minuten bis 120 Minuten, vorzugsweise im Bereich von 2 Minuten bis 60 Minuten.

4. Das Verfahren nach einem der vorherigen Ansprüche, wobei die Kristallisation des BHET-Dimers aus der Lösung im Wesentlichen ohne dem Bilden von BHET-Kristallen durchgeführt wird.

5. Das Verfahren nach einem der vorherigen Ansprüche, wobei das kristallisierte BHET in fester Form rückgewonnen wird, und wobei das Verfahren vorzugsweise ferner den Schritt des Waschens der BHET-Kristalle und/oder des Trocknens der BHET-Kristalle umfasst.

6. Das Verfahren nach Anspruch 5, wobei das Abtrennen des kristallisierten BHET-Dimers mittels einer Filtrationseinheit durchgeführt wird, und wobei die Weiterverarbeitung des abgetrennten kristallisierten BHET-Dimers das Entfernen des abgetrennten kristallisierten BHET-Dimers aus der Filtrationseinheit mittels eines Ethylenglykol aufweisenden Spülstroms umfasst.

7. Das Verfahren nach Anspruch 6, wobei dieser Strom verarbeitet wird, um das BHET-Dimer in BHET umzuwandeln.

8. Das Verfahren nach einem der vorherigen Ansprüche, wobei der Schritt des Depolymerisierens das Steuern eines Molverhältnisses zwischen dem Terephthalatpolymer und Ethylenglykol umfasst, um in dem depolymerisierten Gemisch das Molverhältnis zwischen BHET-Monomer und BHET-Dimer zu steuern, wobei das Molverhältnis vorzugsweise so gesteuert wird, dass mindestens 10 Gew.-% Dimer erhalten werden.

9. Das Verfahren nach einem der vorherigen Ansprüche, wobei die erste Mutterflüssigkeit einer adsorptiven Behandlung unterzogen wird, um aus der ersten Mutterflüssigkeit gelöstes BHET-Dimer zu entfernen, nachdem das kristallisierte BHET-Dimer von der ersten Mutterflüssigkeit abgetrennt wurde und bevor BHET-Kristalle gebildet werden.

10. Das Verfahren nach einem der vorherigen Ansprüche, wobei der Schritt des Depolymerisierens mittels eines das Element Fe aufweisenden Katalysators durchgeführt wird, und wobei das depolymerisierte Gemisch das Element Fe aufweist, in einer Konzentration von mindestens 5 ppm in Bezug auf die Gewichtskonzentration von Bis(2-hydroxyethyl)terephthalat (BHET) des depolymerisierten Gemisches.

11. Ein Reaktorsystem zum Depolymerisieren eines Terephthalat-Polymers zu wiederverwendbarem Rohmaterial, wobei das Reaktorsystem aufweist:
- eine Depolymerisationsstufe, mit zumindest einem Einlass für einen Strom von terephthalathaltigem Polymer und einen Strom von Ethylenglykol; wobei die Depolymerisationsstufe so ausgebildet ist, dass sie das terephthalathaltige Polymer unter Verwendung des Ethylenglykols zu einem depolymerisierten Gemisch depolymerisiert, wobei das depolymerisierte Gemisch zumindest ein Monomer und zumindest ein Dimer aufweist, wobei das zumindest eine Monomer Bis(2-hydroxyethyl)terephthalat (BHET) umfasst und das zumindest eine Dimer das Dimer von Bis(2-hydroxyethyl)terephthalat (BHET-Dimer) umfasst;
- einen ersten Separator, der mit einem Auslass versehen ist, wobei der erste Separator für die Trennung von festen Verbindungen des depolymerisierten Gemisches ausgebildet ist, welches eine Zusammensetzung aufweist, die das zumindest eine Dimer und das zumindest eine Monomer als gelöste Stoffe in einem Gemisch aus Ethylenglykol und Wasser umfasst;
- eine Dimer-Kristallisationseinheit zur Kristallisation des Dimers aus der Zusammensetzung, wobei die verbleibende Zusammensetzung eine erste Mutterflüssigkeit bildet, wobei zur Abtrennung des kristallinen Dimers aus der ersten Mutterflüssigkeit ein Separator angeordnet ist;
- eine Monomer-Kristallisationseinheit, die für die Kristallisation des Monomers aus der ersten Mutterflüssigkeit angeordnet ist; und
- eine Monomer-Kristallisations-Rückgewinnungsstufe, welche stromabwärtig der Monomer-Kristallisationseinheit angeordnet ist.

12. Feste Zusammensetzung, aufweisend zumindest 70 Gew.-% Dimer von Bis(2-hydroxyethyl)terephthalat (BHET) in kristalliner Form, wobei die feste Zusammensetzung vorzugsweise zumindest 80 Gew.-% Dimer von BHET umfasst.

13. Ein Verfahren zum Reinigen einer Bis(2-hydroxyethyl)terephthalat (BHET) enthaltenden Rohzusammensetzung, aufweisend die folgenden Schritte:
a) Bereitstellen der Rohzusammensetzung, welche BHET und Dimere von Bis(2-hydroxyethyl)terephthalat (BHET-Dimer) als gelöste Stoffe in einem Gemisch aus Ethylenglykol und Wasser enthält;
b) Vorbehandeln der Rohzusammensetzung, um eine erste Mutterflüssigkeit mit einem Gehalt an BHET-Dimeren von höchstens 3 Gew.-%, bezogen auf den BHET-Gehalt, zu erhalten;
c) Bilden von BHET-Kristallen aus der ersten Mutterflüssigkeit, wodurch ein Gemisch aus BHET-Kristallen und einer zweiten Mutterflüssigkeit erhalten wird, und
d) Abtrennen der BHET-Kristalle von der zweiten Mutterflüssigkeit, vorzugsweise durch Filtration; und
e) gegebenenfalls ferner den Schritt des Trocknens der abgetrennten BHET-Kristalle.

14. Das Verfahren nach Anspruch 13, wobei die Vorbehandlung der Rohzusammensetzung die Kristallisation von BHET-Dimeren und vorzugsweise die Abtrennung der kristallisierten BHET-Dimere von der verbleibenden ersten Mutterflüssigkeit umfasst.

15. Das Verfahren nach einem der Ansprüche 13 bis 14, wobei der Gehalt an BHET-Dimeren in der ersten Mutterflüssigkeit höchstens 2,0 Gew.-%, bezogen auf BHET, beträgt.

## Revendications

1. Procédé de dépolymérisation d'un polymère de téréphtalate en une matière première réutilisable, le procédé comprenant les étapes consistant à :
a) dépolymériser un polymère de téréphtalate en utilisant de l'éthylène glycol en un mélange dépolymérisé comprenant au moins un monomère et au moins un dimère, dans lequel ledit au moins un monomère comprend du bis(2-hydroxyéthyl)téréphtalate (BHET), et ledit au moins un dimère comprend un dimère de bis(2-hydroxyéthyl)téréphtalate (dimère de BHET),
b) éliminer les composés solides dudit mélange dépolymérisé dans un premier séparateur afin d'obtenir une composition comprenant l'au moins un dimère et l'au moins un monomère en tant que solutés dans un mélange d'éthylène glycol et d'eau ;
c) cristalliser un dimère de BHET à partir de ladite solution, permettant ainsi d'obtenir un mélange de cristaux de dimère de BHET et d'un premier liquide mère ;
d) séparer le dimère de BHET cristallisé du premier liquide mère, puis :
e) former des cristaux de BHET à partir dudit premier liquide mère, permettant ainsi d'obtenir un mélange de cristaux de BHET et d'un deuxième liquide mère ; et
f) récupérer des cristaux de BHET.

2. Procédé selon la revendication 1, dans lequel après l'étape consistant à cristalliser un dimère de BHET et avant l'étape consistant à former des cristaux de BHET, un anti-solvant est ajouté au premier liquide mère pour améliorer la formation de cristaux de BHET à partir du premier liquide mère.

3. Procédé selon l'une quelconque des revendications précédentes, l'étape consistant à cristalliser un dimère de BHET comprenant le contrôle d'au moins un parmi :
i. une concentration en dimère de BHET dans la solution au début de l'étape consistant à cristalliser un dimère de BHET ; et
ii. un rapport volumique entre l'eau et l'éthylène glycol dans la solution au cours de l'étape consistant à cristalliser un dimère de BHET ; et
iii. le contrôle de la solution à une première température T₁ pendant une période située dans la plage de 2 minutes à 120 minutes, de préférence dans la plage de 2 minutes à 60 minutes.

4. Procédé selon l'une quelconque des revendications précédentes, dans lequel la cristallisation du dimère de BHET à partir de ladite solution est réalisée sensiblement sans formation de cristaux de BHET.

5. Procédé selon l'une quelconque des revendications précédentes, dans lequel le BHET cristallisé est récupéré sous forme solide, et dans lequel de préférence le procédé comprend en outre au moins l'une des étapes consistant à laver des cristaux de BHET et à sécher des cristaux de BHET.

6. Procédé selon la revendication 5, dans lequel la séparation du dimère de BHET cristallisé est réalisée au moyen d'une unité de filtration et dans lequel un autre traitement du dimère de BHET cristallisé séparé comprend l'élimination du dimère de BHET cristallisé séparé de l'unité de filtration au moyen d'un jet de rinçage, qui comprend de l'éthylène glycol.

7. Procédé selon la revendication 6, dans lequel ledit jet est traité pour convertir le dimère de BHET en BHET.

8. Procédé selon l'une quelconque des revendications précédentes, dans lequel l'étape consistant à dépolymériser comprend le contrôle d'un rapport molaire entre ledit polymère de téréphtalate et l'éthylène glycol pour contrôler le rapport molaire entre le monomère de BHET et le dimère de BHET dans le mélange dépolymérisé, dans lequel de préférence ledit rapport molaire est contrôlé afin d'obtenir au moins 10 % en poids de dimère.

9. Procédé selon l'une quelconque des revendications précédentes, dans lequel le premier liquide mère est soumis à un traitement d'adsorption pour éliminer le dimère de BHET dissous du premier liquide mère après séparation du dimère de BHET cristallisé du premier liquide mère et avant la formation de cristaux de BHET.

10. Procédé selon l'une quelconque des revendications précédentes, dans lequel l'étape consistant à dépolymériser est réalisée au moyen d'un catalyseur comprenant l'élément Fe, et dans lequel le mélange dépolymérisé comprend l'élément Fe à une concentration d'au moins 5 ppm par rapport à la concentration en poids de bis(2-hydroxyéthyl)téréphtalate (BHET) dans le mélange dépolymérisé.

11. Système de réacteur pour la dépolymérisation d'un polymère de téréphtalate en une matière première réutilisable, ledit système de réacteur comprenant :
- un étage de dépolymérisation comprenant au moins une entrée pour un flux de polymère contenant du téréphtalate et d'un flux d'éthylène glycol ; dans lequel ledit étage de dépolymérisation est configuré pour dépolymériser le polymère contenant du téréphtalate en un mélange dépolymérisé en utilisant l'éthylène glycol, dans lequel ledit mélange dépolymérisé comprend au moins un monomère et au moins un dimère, dans lequel ledit au moins un monomère et au moins un dimère, dans lequel ledit au moins un monomère comprend du bis(2-hydroxyéthyl)téréphtalate (BHET), et ledit au moins un dimère comprend un dimère de bis(2-hydroxyéthyl)téréphtalate (dimère de BHET) ;
- un premier séparateur doté d'une sortie, ledit premier séparateur étant configuré pour la séparation des composés solides dans le mélange dépolymérisé d'une composition comprenant l'au moins un dimère et l'au moins un monomère en tant que solutés dans un mélange d'éthylène glycol et d'eau ;
- une unité de cristallisation de dimère pour la cristallisation de dimère à partir de ladite composition, dans lequel la composition restante constitue un premier liquide mère, dans lequel un séparateur est agencé pour la séparation dudit dimère cristallin dudit premier liquide mère ;
- une unité de cristallisation de monomère agencée pour la cristallisation de monomère à partir dudit premier liquide mère ; et
- un étage de récupération de cristaux de monomère agencé en avant de l'unité de cristallisation de monomère.

12. Composition solide comprenant au moins 70 % en poids de dimère de bis(2-hydroxyéthyl)téréphtalate (BHET) sous forme cristalline, dans lequel la composition solide comprend de préférence au moins 80 % en poids de dimère de BHET.

13. Procédé de purification d'une composition brute comprenant du bis(2-hydroxyéthyl)téréphtalate (BHET), ledit procédé comprend les étapes consistant à :
a) fournir la composition brute qui comprend du BHET et le dimère de bis(2-hydroxyéthyl)téréphtalate (dimère de BHET) en tant que solutés dans un mélange d'éthylène glycol et d'eau ;
b) prétraiter ladite composition brute afin d'obtenir un premier liquide mère ayant une teneur en dimère de BHET d'au maximum 3 % en poids, par rapport à la teneur en BHET ;
c) former des cristaux de BHET à partir du premier liquide mère, permettant ainsi d'obtenir un mélange de cristaux de BHET et un deuxième liquide mère et ;
d) séparer des cristaux de BHET dudit deuxième liquide mère, de préférence au moyen d'une filtration ; et
e) comprenant en outre facultativement l'étape consistant à séchage des cristaux de BHET séparés.

14. Procédé selon la revendication 13, dans lequel le prétraitement de la composition brute comprend la cristallisation de dimère de BHET, et de préférence la séparation du dimère de BHET cristallisé du premier liquide mère restant.

15. Procédé selon l'une quelconque des revendications 13 et 14, dans lequel ladite teneur en dimère de BHET dans le premier liquide mère est au maximum de 2,0 % en poids, par rapport au BHET.
